# EUROPÄISCHE PATENTSCHRIFT

(11) **EP 0 820 252 B1**
(45) Veröffentlichungstag und Bekanntmachung des Hinweises auf die Patenterteilung: **07.07.1999**
(21) Anmeldenummer: 96908912.7
(22) Anmeldetag: 09.04.1996
(51) Int. Cl.: A61B 5/0408

(54) **VERFAHREN ZUR HERSTELLUNG EINER ELEKTRODE ZUM AUFBRINGEN AUF DIE HAUT**
PROCESS FOR MAKING AN ELECTRODE TO BE APPLIED TO THE SKIN
PROCEDE DE PRODUCTION D'UNE ELECTRODE A APPLIQUER SUR LA PEAU

(30) Priorität: 13.04.1995 AT 643955
(43) Veröffentlichungstag der Anmeldung: 28.01.1998
(73) Patentinhaber: Lang, Burrhus, 6020 Innsbruck (AT)
(72) Erfinder: Lang, Burrhus, 6020 Innsbruck (AT)
(74) Vertreter: Torggler, Paul Norbert
(86) Internationale Anmeldenummer: AT9600068
(87) Internationale Veröffentlichungsnummer: WO9632057

(56) Entgegenhaltungen:
- EP-A- 0 635 239
- WO-A-93/00857
- DD-A- 298 616
- US-A- 4 687 137
- US-A- 5 264 249

## Beschreibung

Die Erfindung betrifft ein Verfahren sowie eine Einrichtung zur Herstellung einer Elektrode zum Aufbringen auf die Haut, bei dem ein mit einer Öffnung versehener Träger auf der der Haut abgewandten mit einem die Öffnung überdeckenden Etikett verklebt wird.

Elektroden zum Aufbringen auf die Haut (beispielsweise EKG-Elektroden etc.), welche einen Träger mit einer zentralen Öffnung aufweisen, welche oben von einer Abdeckung (sogenanntes Etikett) abgedeckt ist, sind bereits seit langem bekannt. Üblicherweise weist der Träger auf der Unterseite einen hautfreundlichen Kleber auf, der selbstklebend ist und zum Transport bzw. zur Lagerung durch eine Abziehfolie abgedeckt ist. Nach Abziehen der Abziehfolie kann der Träger auf die Haut des Patienten aufgeklebt werden. Das Etikett trägt einen elektrischen Anschluß, der den Anschluß eines Elektrodenkabels erlaubt, wobei dieser außenliegende Anschluß (Kontaktbereich) elektrisch mit dem Bereich der Öffnung des Trägers verbunden ist, der den eigentlichen Sensorbereich darstellt. In diesem Sensorbereich ist beispielsweise ein flüssiges Elektrolyt-Gel in einem Schwamm oder ein festes leitfähiges Gel angebracht, um die elektrischen Ströme zwischen der Haut und dem eigentlichen Sensorbereich weiterzuleiten. Von dort gelangen dann die Ströme durch das Etikett oder entlang dem Etikett nach außen zum Kontaktbereich.

Es ist bereits bekannt (EP-A-635 239), das Etikett mittels eines aktivierbaren (also zunächst nichtklebenden) Klebers auf dem Träger festzukleben. Beispielsweise kann ein thermoaktivierbarer Kleber verwendet werden. Vor allem bei Elektroden, bei welchen der Kontaktbereich an einem freien Vorsprung des Etiketts gegenüber dem zentralen Sensorbereich seitlich versetzt angeordnet ist, besteht das Grundproblem, daß einerseits das Etikett fest und überall dicht am Träger klebend haften soll und daß andererseits durch das Etikett oder entlang dem Etikett eine gute elektrische Verbindung zwischen dem Sensorbereich und dem seitlich versetzten Kontaktbereich gegeben sein soll. Dabei ist immer zu beachten, daß derartige Elektroden Wegwerfartikel sind und daher im Aufbau einfach und bei der Produktion damit kostengünstig sein sollen.

Um diese Probleme zufriedenstellend zu lösen, sieht das erfindungsgemäße Verfahren vor, daß auf das Etikett und/oder den Träger lediglich auf einem lokal begrenzten Teilbereich ihrer einander zugewandten Oberfläche Kleber aufgetragen wird und dann Etikett und Träger miteinander verklebt werden, wobei ein im Bereich der Öffnung liegenden Bereich des Etiketts vom Kleber frei bleibt.

Es besteht damit insbesondere die Möglichkeit, den Kleber nur im Überlappungsbereich zwischen Etikett und Träger unter Aussparung jenes Teilbereiches des Etiketts, der später bei der Öffnung des Trägers zu liegen kommt, aufzutragen. Damit ist es beispielsweise möglich, ein elektrisch leitfähiges Etikett zu verwenden, welches im Bereich der Öffnung des Trägers einen Sensorbereich ausbildet, der elektrisch mit einem seitlich versetzten, vorzugsweise an einem Vorsprung des Etiketts ausgebildeten, Kontaktbereich verbunden ist. Durch die lokal begrenzte Kleberauftragung kann im Gegensatz zu einer bekannten vollflächigen Kleberauftragung vermieden werden, daß das Etikett auch im Bereich der Öffnung des Trägers vom Kleber abgedeckt wird.

Vielmehr bleibt der Sensorbereich vom Kleber frei und kann somit leicht, beispielsweise über ein leitfähiges Gel, die Haut des Patienten kontaktieren.

Es bestünde grundsätzlich auch die Möglichkeit, das Etikett aus elektrisch isolierendem Material herzustellen und darauf eine schichtförmige elektrisch leitende Leiterbahn anzubringen. Bei dieser Ausführungsform könnte das elektrisch isolierende Etikett vor dem Aufbringen der Leiterbahn bereits mit einem aktivierbaren Kleber beschichtet sein oder überhaupt aus einem klebend aktivierbaren Material (beispielsweise einen Thermoplasten) bestehen. Ein solches Etikett kann dann durch Aktivieren des Klebers (beispielsweise durch Erwärmen eines Schmelzklebers oder durch chemisches Aktivieren eines chemisch aktivierbaren Klebers oder durch UV-Bestrahlung eines UV-aktivierbaren Klebers) auf den Träger aufgeklebt werden, wobei die zwischen Kleberschicht und Träger verlaufende Leiterbahn einen guten elektrischen Kontakt zwischen dem Sensorbereich im Bereich der Öffnung des Trägers und einem seitlich versetzten Kontaktbereich zum Anschluß eines Elektrodenkabels sicherstellt. Mit dem erfindungsgemäßen Verfahren kann nun zusätzlich im Bereich der Leiterbahn lokal begrenzt ein Kleber aufgebracht werden um sicherzustellen, daß auch im Bereich der Leiterbahn zwischen Etikett und Träger ein dichter Abschluß gegeben ist, womit insbesondere ein im Bereich der Öffnung des Trägers vorgesehenes Gel nicht zwischen Etikett und Elektrode unter der Leiterbahn seitlich austreten kann.

Weitere Vorteile und Einzelheiten der Erfindung werden anhand der nachfolgenden Figurenbeschreibung näher erläutert.
Die Fig. 1 zeigt schematisch eine Einrichtung gemäß der Erfindung zur Durchführung eines Ausführungsbeispieles des erfindungsgemäßen Verfahrens,
die Fig. 2 zeigt im Schnitt eine nach dem erfindungsgemäßen Verfahren bzw. mit einer erfindungsgemäßen Einrichtung hergestellte Elektrode,
die Fig. 3 zeigt die Unteransicht eines Etiketts, welches beim erfindungsgemäßen Verfahren bzw. der erfindungsgemäßen Einrichtung eingesetzt werden kann,
die Fig. 4 zeigt ein weiteres Ausführungsbeispiel einer erfindungsgemäßen Einrichtung zur Herstellung einer auf die Haut aufklebbaren Elektrode.

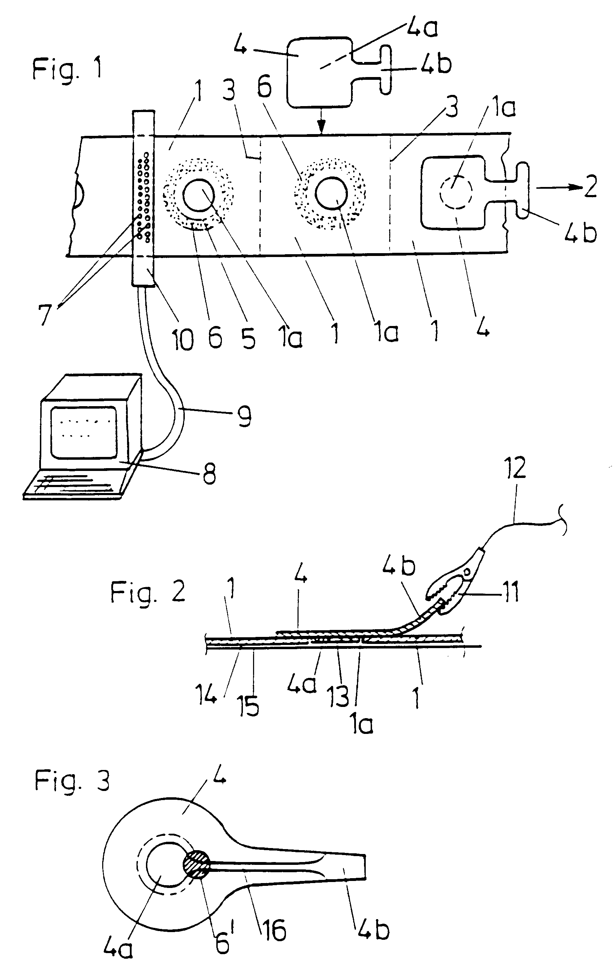

Bei dem in Fig. 1 dargestellten Ausführungsbeispiel wird eine Bahn von Trägern 1 in Richtung des Pfeiles 2 bewegt. Aus dieser Bahn werden dann später in an sich bekannter Weise entlang der Trennungslinien 3 oder durch Ausstanzen die einzelnen Elektroden gebildet. Zunächst handelt es sich aber um eine durchgehende Bahn, die sich leicht über an sich bekannte und daher nicht dargestellte Vorrichtungen zum Transport bewegen läßt.

Ziel des erfindungsgemäßen Verfahrens ist es, ein Etikett 4 auf jeden Träger 1 aufzukleben, und zwar oberhalb einer Öffnung 1a im Träger 1, die beispielsweise vorher in einem nicht gezeigten Stanzprozeß ausgestanzt werden kann. Letztlich soll das Etikett 4 an seiner Unterseite im Bereich der Öffnung 1a des Trägers 1 einen elektrischen Sensorbereich 4a ausbilden, der elektrisch mit dem seitlich versetzten Kontaktbereich 4b zum Anschluß eines Elektrodenkabels verbunden ist. Gleichzeitig soll das Etikett 4 fest und dicht mit dem Träger 1 verbunden sein.

Zu diesem Zweck ist beim Ausführungsbeispiel der Fig. 1 ein elektrisch leitfähiges Etikett 4 vorgesehen und es wird gemäß der Grundidee der Erfindung der Kleber 5 nur in einem lokal begrenzten Teilbereich auf den Träger 1 aufgetragen. Beim dargestellten Ausführungsbeispiel erfolgt der Kleberauftrag ringförmig um die Öffnung 1a herum, also lediglich auf einem Teil des Überlappungsbereiches zwischen Etikett 4 und Träger 1. Durch diesen ringförmigen Auftrag des Klebers wird rundum die Öffnung 1a herum eine dichte Abdichtung zwischen Etikett 4 und Träger 1 erzielt.

Grundsätzlich könnte der lokal begrenzte Kleberauftrag auch auf der Unterseite der Etiketten 4 erfolgen. Wesentlich ist dabei aber, daß die elektrisch leitende Unterseite des Etiketts in jenem Bereich, der später im Bereich der Öffnung 1a des Trägers 1 zu liegen kommt, nicht durch Kleber abgedeckt wird, weil sonst der elektrische Kontakt des Sensorbereichs des Etiketts zur Haut unterbrochen wäre.

Zum lokal begrenzten Kleberauftrag sind beim Ausführungsbeispiel der Fig. 1 mehrere in ihrer zeitlichen Öffnungsdauer gesteuerte Düsen 7 vorgesehen, an denen die Trägerbahn vorbeibewegt wird. Die Düsen sind beim gezeigten Ausführungsbeispiel in zwei feststehenden, gegeneinander versetzten Zeilen von Düsen angeordnet, welche quer zur Bewegungsrichtung 2 der Trägerbahn liegen. Die programmierbare elektronische Steuereinheit 8 steuert über einen Steuerbus die Öffnungsdauer und Öffnungszeit der Düsen der Kleberauftragsvorrichtung 10. Durch geeignete Abstimmung der Öffnungszeit und Öffnungsdauer kann man bei Kenntnis der Geschwindigkeit der Bahn der Träger 1 mittels der Düsen präzise lokal begrenzte Klebermuster auf die Träger auftragen, beispielsweise im vorliegenden Fall einen ringförmig die Öffnung 1a umgebenden Kleberauftrag. Die einzelnen Kleberpunkte können dabei so benachbart liegen, daß sie miteinander verschmelzen und insgesamt einen geschlossenen Kleberring (Teilbereich 6) ergeben. Auf diesen Teilbereich 6 wird dann das beispielsweise seitlich zugeführte Etikett 4 aufgeklebt und festgedrückt. Daraufhin können die einzelnen Träger 1 aus der Trägerbahn ausgeschnitten bzw. ausgestanzt werden, um die einzelnen Elektroden zu bilden.

Die Düsen können auch diagonal zur Laufrichtung liegen. Mit einer diagonalen Anordnung erzielt man bei gleichem Abstand der Düsen voneinander eine größere Nähe der Klebstofftröpfchen aus benachbarten Düsen zueinander.

Der Kleberauftrag erfolgt beim erfindungsgemäßen Verfahren in der Produktionsanlage selbst (also "in line"), nämlich möglichst knapp vor der Verbindung der Etiketten 4 mit den Trägern 1. Damit ist auch möglich, daueraktiven Kleber aufzutragen. Grundsätzlich können aber auch nicht daueraktive, also aktivierbare Kleber, wie z. B. Schmelzkleber aufgetragen werden.

In Fig. 2 ist ein Ausführungsbeispiel einer Elektrode dargestellt, die mit dem erfindungsgemäßen Verfahren hergestellt ist. Der Träger 1 weist in an sich bekannter Weise auf seiner Unterseite eine selbstklebende hautfreundliche Schicht 14 auf, die von einer Abdeckfolie 15 zu Transport- und Lagerzwecken abgedeckt ist. Diese Abdeckfolie 15 deckt auch ein elektrisch leitendes Gel 13 ab, das im Bereich der Öffnung 1a des Trägers 1 vorgesehen ist. Auf den Träger 1 ist auf der Oberseite mittels lokal aufgebrachtem Kleber ein Etikett 4 festgeklebt, das aus elektrisch leitendem Material besteht. Das Etikett 4 hat gegenüber dem mittleren Sensorbereich 4a, der über das Gel 13 mit der Haut in Kontakt steht, einen seitlich versetzten, frei vorstehenden Vorsprung (Lappen 4b), an den beispielsweise über eine Krokodilklemme 11 ein Elektrodenkabel 12 anschließbar ist.

Wie bereits erwähnt, kann das Etikett 4 vollständig aus elektrisch leitfähigem Material sein, wobei nicht nur Metalle, sondern auch elektrisch leitende Kunststoffe oder beispielsweise Carbon in Frage kommen. Es ist aber auch möglich, daß das Etikett 4 aus einem elektrisch isolierenden Material besteht und auf der gesamten Unterseite, die dem Träger 1 zugewandt ist, mit leitfähigem Material versehen, beispielsweise beschichtet ist. In jedem Fall wird durch den lokal begrenzten Kleberauftrag verhindert, daß der Sensorbereich 4a durch Kleber abgedeckt wird und damit seine Leitfähigkeit zur Haut hin verliert.

Fig. 3 zeigt ein weiteres Ausführungsbeispiel eines Etiketts, das mittels des erfindungsgemäßen Verfahrens mit einem Träger verbunden ist. Die Fig. 3 zeigt eine Unteransicht auf das Etikett, also jene Seite, die dann auf der Oberseite des Trägers zu liegen kommt. Auf dieser Unterseite des Etiketts 4 ist zunächst eine aktivierbare, beispielsweise thermoaktivierbare Kleberschicht vollständig vorgesehen. Auf diese Schicht aufgedruckt ist eine elektrische schichtförmige Leiterbahn 16, die einerseits einen Sensorbereich 4a und andererseits einen Kontaktbereich 4b ausbildet. Nachdem das Etikett auf den Träger aufgelegt worden ist, kann beispielsweise durch Wärmezufuhr die Verklebung mit dem Träger erfolgen. Dabei würde aber im Bereich der Leiterbahn 16 keine Verklebung erfolgen. Um hier doch eine Verklebung zu erzielen, ist vorgesehen, im eng begrenzten Teilbereich 6' lokal einen Kleber zusätzlich aufzubringen, der die Leiterbahn 16 zum Träger hin abdichtet.

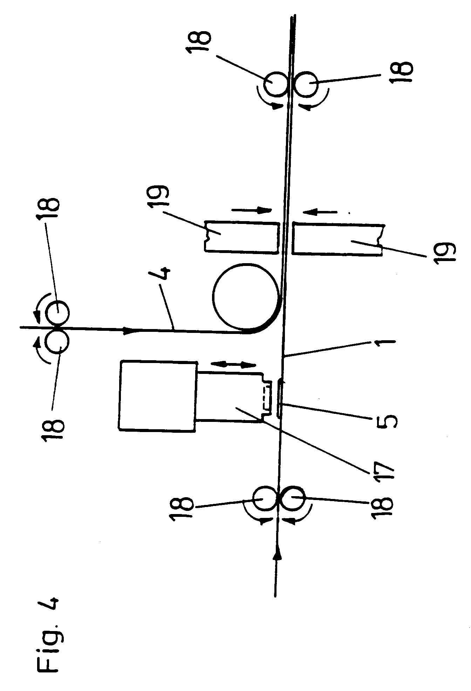

Die Fig. 4 zeigt ein Ausführungsbeispiel einer erfindungsgemäßen Einrichtung, bei der anstelle von Düsen eine Tampon-Druckeinheit 17 vorgesehen ist, um Kleber 5 auf lokal begrenzten Bereichen auf die Bahn der Träger 1 aufzustempeln. Sowohl die Bahn der Träger 1 wie die Bahn der Etiketten 4 wird über Transportvorrichtungen 18, die an sich bekannt sind, angetrieben. Nach der Kleberauftragseinheit 17 erfolgt in einer Fügestation 19 das Zusammenfügen von Etiketten 4 mit Trägern 1. Anschließend können die Elektroden aus der Bahn ausgestanzt bzw. abgeschnitten werden.

## Patentansprüche

1. Verfahren zur Herstellung einer Elektrode zum Aufbringen auf die Haut, bei dem ein mit einer Öffnung (1a) versehener Träger (1) auf der der Haut abgewandten Seite (11) mit einem die Öffnung überdeckenden Etikett (4) verklebt wird, dadurch gekennzeichnet, daß auf das Etikett (4) und/oder den Träger (1) lediglich auf einem lokal begrenzten Teilbereich (6) ihrer einander zugewandten Oberfläche Kleber (5) aufgetragen wird und dann Etikett und Träger miteinander verklebt werden, wobei ein im Bereich der Öffnung liegender elektrisch leitender Bereich (4a) des Etiketts vom Kleber frei bleibt.

2. Verfahren nach Anspruch 1, dadurch gekennzeichnet, daß das Etikett und/oder der Träger lediglich auf einem Teil des Überlappungsbereiches zwischen Etikett und Träger mit Kleber versehen wird.

3. Verfahren nach Anspruch 1 oder 2, dadurch gekennzeichnet, daß jener Teilbereich des Etiketts, der später bei der Öffnung des Trägers zu liegen kommt, von der Klebeauftragung ausgespart bleibt.

4. Verfahren nach einem der Ansprüche 1 bis 3, dadurch gekennzeichnet, daß ein elektrisch leitfähiges Etikett verwendet wird, welches vorzugsweise einen im Bereich der Öffnung des Trägers liegenden Sensorbereich elektrisch mit einem seitlich versetzten, an einem Vorsprung des Etiketts ausgebildeten Kontaktbereich verbindet.

5. Verfahren nach Anspruch 4, dadurch gekennzeichnet, daß das Etikett aus leitfähigem Material besteht oder auf der gesamten Unterseite, die dem Träger zugewandt ist, mit leitfähigem Material versehen ist.

6. Verfahren nach Anspruch 4, dadurch gekennzeichnet, daß das Etikett aus elektrisch isolierendem Material besteht, auf das eine schichtförmige, elektrisch leitende Leiterbahn aufgebracht, vorzugsweise aufgedruckt ist.

7. Verfahren nach Anspruch 5 oder 6, dadurch gekennzeichnet, daß auf dem Träger und/oder dem Etikett ein die Öffnung im Träger ringförmig umgebender Kleberauftrag erfolgt.

8. Verfahren nach Anspruch 6, dadurch gekennzeichnet, daß der Kleber auf die Leiterbahn und vorzugsweise im wesentlichen nur auf diese aufgebracht wird.

9. Verfahren nach einem der Ansprüche 1 bis 8, dadurch gekennzeichnet, daß der lokal begrenzte Kleberauftrag über mehrere in ihrer zeitlichen Öffnungsdauer gesteuerte Düsen erfolgt, wobei das Etikett und/oder der Träger einerseits und die Düsen andererseits relativ zueinander bewegt werden.

10. Verfahren nach Anspruch 9, dadurch gekennzeichnet, daß die Düsen, vorzugsweise in Form von einer oder mehreren feststehenden Zeilen von Düsen, quer zur Bewegungsrichtung zwischen Düsen und Etikett bzw. Träger über die gesamte Breite des gewünschten Kleberauftrages verteilt angeordnet werden.

11. Verfahren nach Anspruch 9 oder 10, dadurch gekennzeichnet, daß die Öffnungszeitpunkte und Öffnungsdauern der einzelnen Düsen von einer programmier baren elektronischen Steuereinheit gesteuert werden.

12. Verfahren nach einem der Ansprüche 1 bis 8, dadurch gekennzeichnet, daß der lokal begrenzte Kleberauftrag durch Aufdrucken des Klebers, beispielsweise im Siebdruck- oder Tampondruckverfahren erfolgt.

13. Verfahren nach einem der Ansprüche 1 bis 8, dadurch gekennzeichnet, daß der Kleberauftrag mittels Klischee und Tauchwalze oder Klischee und Tampondruck erfolgt.

14. Verfahren nach einem der Ansprüche 1 bis 13, dadurch gekennzeichnet, daß ein daueraktiver Kleber oder ein aktivierbarer Kleber, beispielsweise ein thermoaktivierbarer Kleber, verwendet wird.

15. Einrichtung zur Herstellung von Elektroden zum Aufbringen auf die Haut, bei der ein mit einer Öffnung versehener Träger auf der der Haut abgewandten Seite mit einem die Öffnung überdeckenden Etikett verklebt wird, mit Vorrichtungen zum Transport von vorzugsweise in Bahnen vorgesehenen Trägern und Etiketten, gekennzeichnet durch eine Kleberauftragseinheit (17) zum lokal begrenzten Auftragen von Kleber auf die Träger (1) und/oder Etiketten (4).

16. Einrichtung nach Anspruch 15, dadurch gekennzeichnet, daß die Kleberauftragseinheit (17) knapp vor einer Fügestation (19) angeordnet ist, in der jeweils ein Etikett (4) bzw. ein entsprechender Abschnitt einer Etikettenbahn mit einem Träger (1) bzw. einem entsprechenden Abschnitt einer Trägerbahn zusammengefügt werden.

17. Einrichtung nach Anspruch 15 oder 16, dadurch gekennzeichnet, daß die Kleberauftragsvorrichtung mehrere feststehende, in ihrer zeitlichen Öffnungsdauer gesteuerte Düsen (7) umfaßt, über die Kleber lokal gezielt auf die vorbeibewegten Etiketten (4) und/oder Träger (1) aufbringbar ist.

18. Einrichtung nach Anspruch 17, dadurch gekennzeichnet, daß die Düsen (7) quer zur Bewegungsrichtung (2) von Etiketten (4) bzw. Trägern (1) in einer oder mehreren Zeilen über die Breite des gewünschten Kleberauftrags verteilt angeordnet sind.

19. Einrichtung nach Anspruch 17 oder 18, gekennzeichnet durch eine programmierbare elektronische Steuereinheit (8) zum Steuern der Öffnungszeitpunkte und Öffnungsdauern der Düsen (7).

20. Einrichtung nach Anspruch 15 oder 16, dadurch gekennzeichnet, daß die Kleberauftragsvorrichtung (17) eine Druckeinheit, vorzugsweise eine Siebdruck- oder eine Tampondruckeinheit aufweist.

## Claims

1. A process for the production of an electrode for application to the skin, in which a carrier (1) provided with an opening (1a) is stuck, on the side remote from the skin, to a tag portion (4) which covers the opening, characterised in that adhesive (5) is applied to the tag portion (4) and / or the carrier (1) only on a locally delimited portion (6) of their mutually facing surface and then the tag portion and the carrier are stuck together, an electrically conductive portion (4a) of the tag lying in the region of the opening remaining free from adhesive.

2. A process as set forth in claim 1 characterised in that the tag portion and/or the carrier is provided with adhesive only on a part of the region of overlap between the tag portion and the carrier.

3. A process as set forth in claim 1 or 2 characterised in that that region of the tag portion which later comes to lie at the opening of the carrier remains free from the application of adhesive.

4. A process as set forth in one of claims 1 through 3 characterised in that an electrically conductive tag portion is used, which preferably electrically connects a sensor region disposed in the region of the opening of the carrier to a laterally displaced contact region provided on a projection of the tag portion.

5. A process as set forth in claim 4 characterised in that the tag portion comprises conductive material or is provided with conductive material on the entire underside which faces towards the carrier.

6. A process as set forth in claim 4 characterised in that the tag portion comprises electrically insulating material to which there is applied, preferably by printing, an electrically conductive conductor track in layer form.

7. A process as set forth in claim 5 or 6 characterised in that an application of adhesive which surrounds the opening in the carrier in an annular configuration is provided on the carrier and/or the tag portion.

8. A process as set forth in claim 6 characterized in that the adhesive is applied to the conductor track and preferably substantially only thereto.

9. A process as set forth in one of claims 1 through 8 characterised in that the locally delimited application of adhesive is effected by way of a plurality of nozzles which are controlled in respect of their period of opening, wherein the tag portion and/or the carrier on the one hand and the nozzles on the other hand are moved relative to each other.

10. A process as set forth in claim 9 characterised in that the nozzles, preferably in the form of one or more stationary rows of nozzles, are arranged distributed over the entire width of the desired application of adhesive, transversely with respect to the direction of movement as between nozzles and tag portion or carrier.

11. A process as set forth in claim 9 or 10 characterised in that the opening times and the periods of opening of the individual nozzles are controlled by a programmable electronic control unit.

12. A process as set forth in one of claims 1 through 8 characterised in that the locally delimited application of adhesive is effected by printing on the adhesive, for example by a screen printing process or a pad printing process.

13. A process as set forth in one of claims 1 through 8 characterised in that the application of adhesive is effected by means of a printing plate and dip cylinder or printing plate and pad print.

14. A process as set forth in one of claims 1 through 13 characterised in that a permanently active adhesive or an activatable adhesive, for example a heat-activatable adhesive, is used.

15. Apparatus for the production of electrodes for application to the skin, in which a carrier provided with an opening is stuck, on the side remote from the skin, to a tag portion which covers over the opening, having devices for transporting tag portions and carriers preferably provided in webs, characterised by an adhesive applicator unit (17) for the locally delimited application of adhesive to the carriers (1) and/or tag portions (4).

16. Apparatus as set forth in claim 15 characterised in that the adhesive applicator unit (17) is arranged closely upstream of a joining station (19) in which a respective tag portion (4) or a corresponding part of a tag portion web is brought together a carrier (1) or a corresponding part of a carrier web.

17. Apparatus as set forth in claim 15 or 16 characterised in that the adhesive applicator arrangement includes a plurality of stationary nozzles (7) which are controlled in respect of their period of opening and by way of which the adhesive can be locally specifically applied to the tag portions (4) and/or carriers (1) which are moved therepast.

18. Apparatus as set forth in claim 17 characterised in that the nozzles (7) are arranged distributed over the width of the desired application of adhesive transversely to the direction of movement (2) of tag portions (4) or carriers (1) in one or more rows.

19. Apparatus as set forth in claim 17 or 18 characterised by a programmable electronic control unit (8) for controlling the opening times and the periods of opening of the nozzles (7).

20. Apparatus as set forth in claim 15 or 16 characterised in that the adhesive applicator arrangement (17) has a printing unit, preferably a screen printing unit or a pad printing unit.

## Revendications

1. Procédé de réalisation d'une électrode à appliquer sur la peau, dans lequel un support (1) muni d'une ouverture (1a) est équipé par collage, sur le coté opposé à la peau, d'une étiquette (4) recouvrant l'ouverture, caractérisé en ce que de la colle (5) est appliquée sur l'étiquette (4) et/ou sur le support (1) uniquement sur une zone partielle (6), limitée localement, de leurs surfaces tournées l'une vers l'autre et en ce que l'étiquette et le support sont ensuite collées ensemble, une zone électroconductrice (4a) de l'étiquette se situant dans la zone de l'ouverture restant libre de colle.

2. Procédé selon la revendication 1, caractérisé en ce que l'étiquette et/ou le support ne sont munis de colle que sur une partie de la zone de recouvrement ente l'étiquette et le support.

3. Procédé selon la revendication 1 ou 2, caractérisé en ce que la zone partielle de l'étiquette se situant ultérieurement au niveau de l'ouverture du support est exempte de l'application de colle.

4. Procédé selon l'une des revendications 1 à 3, caractérisé en ce qu'on utilise une étiquette électroconductrice qui relie électriquement de préférence une zone de détecteur située dans la zone de l'ouverture du support à une zone de contact décalée latéralement et réalisée sur une partie en saillie de l'étiquette.

5. Procédé selon la revendication 4, caractérisé en ce que l'étiquette est réalisée dans un matériau électroconducteur ou en ce qu'elle est pourvue d'un matériau électroconducteur sur l'ensemble de sa partie inférieure dirigée vers le support.

6. Procédé selon la revendication 4, caractérisé en ce que l'étiquette se compose d'un matériau isolant sur lequel est appliquée, de préférence par impression, une bande électroconductrice en forme de couche.

7. Procédé selon la revendication 5 ou 6, caractérisé en ce que sur le support et/ou sur l'étiquette a lieu une application de colle circulaire entourant l'ouverture du support.

8. Procédé selon la revendication 6, caractérisé en ce que la colle est appliquée sur la bande conductrice et de préférence pour l'essentiel uniquement sur celle-ci.

9. Procédé selon l'une des revendications 1 à 8, caractérisé en ce que l'application de colle localement limitée a lieu par l'intermédiaire de plusieurs buses dont le temps d'ouverture est commandé, l'étiquette et/ou le support d'une part et les buses d'autre part faisant l'objet d'un déplacement relatif entre eux.

10. Procédé selon la revendication 9, caractérisé en ce que les buses prenant de préférence la forme d'une ou de plusieurs lignes de buses fixes sont disposées transversalement à la direction de déplacement entre les buses et l'étiquette ou le support, et sont agencées de façon répartie sur l'ensemble de la largeur de l'application de colle souhaitée.

11. Procédé selon la revendication 9 ou 10, caractérisé en ce que les moments et les durées d'ouverture des différentes buses sont commandés par une unité de commande électronique programmable.

12. Procédé selon l'une des revendications 1 à 8, caractérisé en ce que l'application localement limitée de colle a lieu par impression de la colle, par exemple par un procédé de sérigraphie ou d'impression au tampon.

13. Procédé selon l'une des revendications 1 à 8, caractérisé en ce que l'application de la colle a lieu par cliché et cylindre plongeur ou par cliché et impression au tampon.

14. Procédé selon l'une des revendications 1 à 13, caractérisé en ce qu'une colle à activation permanente ou une colle activable, comme par exemple une colle thermoactivable, est utilisée.

15. Dispositif pour la réalisation d'électrodes à appliquer sur la peau, avec lequel un support muni d'une ouverture est équipé par collage, sur le coté opposé à la peau, d'une étiquette recouvrant l'ouverture, et comprenant des dispositifs de transport de supports et d'étiquettes prévus de préférence sous forme de bandes, caractérisé par une unité d'application de colle (17) pour une application localement limitée de colle sur les supports (1) et/ou étiquettes (4).

16. Dispositif selon la revendication 15, caractérisé en ce que l'unité d'application de colle (17) est disposée juste devant une station d'assemblage (19) dans laquelle sont assemblées respectivement une étiquette (4) ou une section correspondante d'une bande d'étiquettes avec un support (1) ou une section correspondante d'une bande de support.

17. Dispositif selon la revendication 15 ou 16, caractérisé en ce que l'unité d'application de colle comprend plusieurs buses fixes (7) à durée d'ouverture commandée, au moyen desquelles la colle peut être appliquée localement et de manière ciblée sur les étiquettes (4) et/ou les supports (1) qui passent devant elles.

18. Dispositif selon la revendication 17, caractérisé en ce que les buses (7) sont disposées transversalement à la direction de déplacement (2) des étiquettes (4) ou des supports (1) en une ou en plusieurs lignes agencées de façon répartie sur la largeur de l'application de colle souhaitée.

19. Dispositif selon la revendication 17 ou 18, caractérisé par une unité de commande électronique programmable (8) pour commander les moments et les durées d'ouverture des buses (7) .

20. Dispositif selon la revendication 15 ou 16, caractérisé en ce que l'unité d'application de colle (17) comprend une unité d'impression, de préférence une unité de sérigraphie ou une unité d'impression au tampon.
